# EUROPEAN PATENT APPLICATION

(11) **EP 2 930 188 A1**
(43) Date of publication of application: **14.10.2015**
(21) Application number: 14164523.4
(22) Date of filing: 13.04.2014
(51) Int. Cl.: C07K 16/28, C07K 16/46

(54) **Trifunctional antigen-binding molecule**

(71) Applicant: Affimed Therapeutics AG, 69120 Heidelberg (DE)
(72) Inventor: Little, Melvyn, 25826 St. Peter Ording (DE); Zhukovsky, Eugene Alexander, 69120 Heidelberg (DE); Eser, Markus, 40593 Düsseldorf (DE); Weichel, Michael, 65474 Bischofsheim (DE); Gantke, Thorsten, 68535 Edingen-Neckarhausen (DE); Reusch, Uwe, 67487 Maikammer (DE); Ellwanger, Kristina, 69120 Heidelberg (DE); Le Gall, Fabrice, 55122 Mainz (DE)
(74) Representative: Schüssler, Andrea

(57) **Abstract**

The invention relates to a trispecific antigen-binding polypeptide dimer comprising a first polypeptide and a second polypeptide, each polypeptide having at least four domains of immunoglobulin chain variable regions linked one after another, wherein two of said four antigen binding sites are specific for the same antigen and the polypeptide can be used as a medicament for tumor therapy.

SEQUENCE LISTING

<110>  Affimed Therapeutics AG

<120>  Trifunctional Antigen Binding Molecule

<130>  A 3256EP

<160>  6

<170>  PatentIn version 3.5

<210>  1
<211>  509
<212>  PRT
<213>  artificial sequence

<220>
<223>  polypeptide chain

<400>  1

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser Gly Gly Ser Gly Gly Ser Gln Val Gln
        115                 120                 125

Leu Gln Gln Ser Gly Ala Glu Leu Ala Arg Pro Gly Ala Ser Val Lys
    130                 135                 140

Met Ser Cys Lys Ala Ser Gly Tyr Thr Phe Thr Arg Tyr Thr Met His
145                 150                 155                 160

Trp Val Lys Gln Arg Pro Gly Gln Gly Leu Glu Trp Ile Gly Tyr Ile

                165                 170                 175

Asn Pro Ser Arg Gly Tyr Thr Asn Tyr Asn Gln Lys Phe Lys Asp Lys
            180                 185                 190

Ala Thr Leu Thr Thr Asp Lys Ser Ser Ser Thr Ala Tyr Met Gln Leu
        195                 200                 205

Ser Ser Leu Thr Ser Glu Asp Ser Ala Val Tyr Tyr Cys Ala Arg Tyr
    210                 215                 220

Tyr Asp Asp His Tyr Cys Leu Asp Tyr Trp Gly Gln Gly Thr Thr Leu
225                 230                 235                 240

Thr Val Ser Ser Gly Gly Ser Gly Gly Ser Gly Gly Ser Gln Val Gln
                245                 250                 255

Leu Gln Gln Ser Gly Ala Glu Leu Ala Arg Pro Gly Ala Ser Val Lys
            260                 265                 270

Met Ser Cys Lys Ala Ser Gly Tyr Thr Phe Thr Thr Tyr Thr Ile His
        275                 280                 285

Trp Val Arg Gln Arg Pro Gly His Asp Leu Glu Trp Ile Gly Tyr Ile
    290                 295                 300

Asn Pro Ser Ser Gly Tyr Ser Asp Tyr Asn Gln Asn Phe Lys Gly Lys
305                 310                 315                 320

Thr Thr Leu Thr Ala Asp Lys Ser Ser Asn Thr Ala Tyr Met Gln Leu
                325                 330                 335

Asn Ser Leu Thr Ser Glu Asp Ser Ala Val Tyr Tyr Cys Ala Arg Arg
            340                 345                 350

Ala Asp Tyr Gly Asn Tyr Glu Tyr Thr Trp Phe Ala Tyr Trp Gly Gln
        355                 360                 365

Gly Thr Thr Val Thr Val Ser Ser Gly Gly Ser Gly Gly Ser Gly Gly
    370                 375                 380

Ser Gly Gly Ser Gly Gly Ser Asp Ile Val Met Thr Gln Ser Pro Lys
385                 390                 395                 400

Phe Met Ser Thr Ser Val Gly Asp Arg Val Thr Val Thr Cys Lys Ala
                405                 410                 415

Ser Gln Asn Val Gly Thr Asn Val Ala Trp Phe Gln Gln Lys Pro Gly
            420                 425                 430

Gln Ser Pro Lys Val Leu Ile Tyr Ser Ala Ser Tyr Arg Tyr Ser Gly
        435                 440                 445

Val Pro Asp Arg Phe Thr Gly Ser Gly Ser Gly Thr Asp Phe Thr Leu
    450                 455                 460

Thr Ile Ser Asn Val Gln Ser Glu Asp Leu Ala Glu Tyr Phe Cys Gln
465                 470                 475                 480

Gln Tyr His Thr Tyr Pro Leu Thr Phe Gly Gly Gly Thr Lys Leu Glu
                485                 490                 495

Ile Asn Ala Ala Ala Gly Ser His His His His His His
            500                 505

<210>  2
<211>  490
<212>  PRT
<213>  artificial sequence

<220>
<223>  polypeptide chain

<400>  2

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Leu Ala Ser Gly Val Pro Ala His Phe Arg Gly Ser
    50                  55                  60

Gly Ser Gly Thr Ser Tyr Ser Leu Thr Ile Ser Gly Met Glu Ala Glu
65                  70                  75                  80

Asp Ala Ala Thr Tyr Tyr Cys Gln Gln Trp Ser Ser Asn Pro Phe Thr
                85                  90                  95

Phe Gly Ser Gly Thr Lys Leu Glu Ile Asn Gly Gly Ser Gly Gly Ser
            100                 105                 110

Gln Ile Val Leu Thr Gln Ser Pro Ala Ile Met Ser Ala Ser Pro Gly

115                 120                 125

Glu Lys Val Thr Met Thr Cys Ser Ala Ser Ser Ser Val Ser Tyr Met
    130                 135                 140

Asn Trp Tyr Gln Gln Lys Ser Gly Thr Ser Pro Lys Arg Trp Ile Tyr
145                 150                 155                 160

Asp Thr Ser Lys Leu Ala Ser Gly Val Pro Ala His Phe Arg Gly Ser
                165                 170                 175

Gly Ser Gly Thr Ser Tyr Ser Leu Thr Ile Ser Gly Met Glu Ala Glu
            180                 185                 190

Asp Ala Ala Thr Tyr Tyr Cys Gln Gln Trp Ser Ser Asn Pro Phe Thr
        195                 200                 205

Phe Gly Ser Gly Thr Lys Leu Glu Ile Asn Gly Gly Ser Gly Gly Ser
    210                 215                 220

Gly Gly Ser Gln Val Gln Leu Gln Gln Ser Gly Ala Glu Leu Val Arg
225                 230                 235                 240

Pro Gly Ser Ser Val Lys Ile Ser Cys Lys Ala Ser Gly Tyr Ala Phe
                245                 250                 255

Ser Ser Tyr Trp Met Asn Trp Val Lys Gln Arg Pro Gly Gln Gly Leu
            260                 265                 270

Glu Trp Ile Gly Gln Ile Trp Pro Gly Asp Gly Asp Thr Asn Tyr Asn
        275                 280                 285

Gly Lys Phe Lys Gly Lys Ala Thr Leu Thr Ala Asp Glu Ser Ser Ser
    290                 295                 300

Thr Ala Tyr Met Gln Leu Ser Ser Leu Ala Ser Glu Asp Ser Ala Val
305                 310                 315                 320

Tyr Phe Cys Ala Arg Arg Glu Thr Thr Thr Val Gly Arg Tyr Tyr Tyr
                325                 330                 335

Ala Met Asp Tyr Trp Gly Gln Gly Thr Ser Val Thr Val Ser Ser Gly
            340                 345                 350

Gly Ser Gly Gly Ser Gly Gly Ser Gly Gly Ser Gly Gly Ser Asp Ile
        355                 360                 365

Leu Leu Thr Gln Thr Pro Ala Ser Leu Ala Val Ser Leu Gly Gln Arg
    370                 375                 380

Ala Thr Ile Ser Cys Lys Ala Ser Gln Ser Val Asp Tyr Asp Gly Asp
385                 390                 395                 400

Ser Tyr Leu Asn Trp Tyr Gln Gln Ile Pro Gly Gln Pro Pro Lys Leu
                405                 410                 415

Leu Ile Tyr Asp Ala Ser Asn Leu Val Ser Gly Ile Pro Pro Arg Phe
            420                 425                 430

Ser Gly Ser Gly Ser Gly Thr Asp Phe Thr Leu Asn Ile His Pro Val
        435                 440                 445

Glu Lys Val Asp Ala Ala Thr Tyr His Cys Gln Gln Ser Thr Glu Asp
    450                 455                 460

Pro Trp Thr Phe Gly Gly Gly Thr Lys Leu Glu Ile Asn Ala Ala Ala
465                 470                 475                 480

Gly Ser Asp Tyr Lys Asp Asp Asp Asp Lys
                485                 490

<210>  3
<211>  513
<212>  PRT
<213>  artificial sequence

<220>
<223>  polypeptide chain

<400>  3

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Thr Tyr
            20                  25                  30

Thr Ile His Trp Val Arg Gln Arg Pro Gly His Asp Leu Glu Trp Ile
        35                  40                  45

Gly Tyr Ile Asn Pro Ser Ser Gly Tyr Ser Asp Tyr Asn Gln Asn Phe
    50                  55                  60

Lys Gly Lys Thr Thr Leu Thr Ala Asp Lys Ser Ser Asn Thr Ala Tyr
65                  70                  75                  80

Met Gln Leu Asn Ser Leu Thr Ser Glu Asp Ser Ala Val Tyr Tyr Cys

85                  90                  95

Ala Arg Arg Ala Asp Tyr Gly Asn Tyr Glu Tyr Thr Trp Phe Ala Tyr
            100                 105                 110

Trp Gly Gln Gly Thr Thr Val Thr Val Ser Ser Gly Gly Ser Gly Gly
        115                 120                 125

Ser Gln Val Gln Leu Gln Gln Ser Gly Ala Glu Leu Val Arg Pro Gly
    130                 135                 140

Ser Ser Val Lys Ile Ser Cys Lys Ala Ser Gly Tyr Ala Phe Ser Ser
145                 150                 155                 160

Tyr Trp Met Asn Trp Val Lys Gln Arg Pro Gly Gln Gly Leu Glu Trp
                165                 170                 175

Ile Gly Gln Ile Trp Pro Gly Asp Gly Asp Thr Asn Tyr Asn Gly Lys
            180                 185                 190

Phe Lys Gly Lys Ala Thr Leu Thr Ala Asp Glu Ser Ser Ser Thr Ala
        195                 200                 205

Tyr Met Gln Leu Ser Ser Leu Ala Ser Glu Asp Ser Ala Val Tyr Phe
    210                 215                 220

Cys Ala Arg Arg Glu Thr Thr Thr Val Gly Arg Tyr Tyr Tyr Ala Met
225                 230                 235                 240

Asp Tyr Trp Gly Gln Gly Thr Ser Val Thr Val Ser Ser Gly Gly Ser
                245                 250                 255

Gly Gly Ser Gly Gly Ser Gln Val Gln Leu Gln Gln Ser Gly Ala Glu
            260                 265                 270

Leu Ala Arg Pro Gly Ala Ser Val Lys Met Ser Cys Lys Ala Ser Gly
        275                 280                 285

Tyr Thr Phe Thr Arg Tyr Thr Met His Trp Val Lys Gln Arg Pro Gly
    290                 295                 300

Gln Gly Leu Glu Trp Ile Gly Tyr Ile Asn Pro Ser Arg Gly Tyr Thr
305                 310                 315                 320

Asn Tyr Asn Gln Lys Phe Lys Asp Lys Ala Thr Leu Thr Thr Asp Lys
                325                 330                 335

Ser Ser Ser Thr Ala Tyr Met Gln Leu Ser Ser Leu Thr Ser Glu Asp
            340                 345                 350

Ser Ala Val Tyr Tyr Cys Ala Arg Tyr Tyr Asp Asp His Tyr Cys Leu
        355                 360                 365

Asp Tyr Trp Gly Gln Gly Thr Thr Leu Thr Val Ser Ser Gly Gly Ser
    370                 375                 380

Gly Gly Ser Gly Gly Ser Gly Gly Ser Gly Gly Ser Gln Ile Val Leu
385                 390                 395                 400

Thr Gln Ser Pro Ala Ile Met Ser Ala Ser Pro Gly Glu Lys Val Thr
                405                 410                 415

Met Thr Cys Ser Ala Ser Ser Ser Val Ser Tyr Met Asn Trp Tyr Gln
            420                 425                 430

Gln Lys Ser Gly Thr Ser Pro Lys Arg Trp Ile Tyr Asp Thr Ser Lys
        435                 440                 445

Leu Ala Ser Gly Val Pro Ala His Phe Arg Gly Ser Gly Ser Gly Thr
    450                 455                 460

Ser Tyr Ser Leu Thr Ile Ser Gly Met Glu Ala Glu Asp Ala Ala Thr
465                 470                 475                 480

Tyr Tyr Cys Gln Gln Trp Ser Ser Asn Pro Phe Thr Phe Gly Ser Gly
                485                 490                 495

Thr Lys Leu Glu Ile Asn Ala Ala Ala Gly Ser His His His His His
            500                 505                 510

His

<210> 4
<211> 486
<212> PRT
<213> artificial sequence

<220>
<223> polypeptide chain

<400> 4

Asp Ile Leu Leu Thr Gln Thr Pro Ala Ser Leu Ala Val Ser Leu Gly
1               5                   10                  15

Gln Arg Ala Thr Ile Ser Cys Lys Ala Ser Gln Ser Val Asp Tyr Asp

20                  25                  30

Gly Asp Ser Tyr Leu Asn Trp Tyr Gln Gln Ile Pro Gly Gln Pro Pro
        35                  40                  45

Lys Leu Leu Ile Tyr Asp Ala Ser Asn Leu Val Ser Gly Ile Pro Pro
    50                  55                  60

Arg Phe Ser Gly Ser Gly Ser Gly Thr Asp Phe Thr Leu Asn Ile His
65                  70                  75                  80

Pro Val Glu Lys Val Asp Ala Ala Thr Tyr His Cys Gln Gln Ser Thr
                85                  90                  95

Glu Asp Pro Trp Thr Phe Gly Gly Gly Thr Lys Leu Glu Ile Asn Gly
            100                 105                 110

Gly Ser Gly Gly Ser Asp Ile Val Met Thr Gln Ser Pro Lys Phe Met
        115                 120                 125

Ser Thr Ser Val Gly Asp Arg Val Thr Val Thr Cys Lys Ala Ser Gln
    130                 135                 140

Asn Val Gly Thr Asn Val Ala Trp Phe Gln Gln Lys Pro Gly Gln Ser
145                 150                 155                 160

Pro Lys Val Leu Ile Tyr Ser Ala Ser Tyr Arg Tyr Ser Gly Val Pro
                165                 170                 175

Asp Arg Phe Thr Gly Ser Gly Ser Gly Thr Asp Phe Thr Leu Thr Ile
            180                 185                 190

Ser Asn Val Gln Ser Glu Asp Leu Ala Glu Tyr Phe Cys Gln Gln Tyr
        195                 200                 205

His Thr Tyr Pro Leu Thr Phe Gly Gly Gly Thr Lys Leu Glu Ile Asn
    210                 215                 220

Gly Gly Ser Gly Gly Ser Gly Gly Ser Gln Val Gln Leu Gln Gln Ser
225                 230                 235                 240

Gly Ala Glu Leu Ala Arg Pro Gly Ala Ser Val Lys Met Ser Cys Lys
                245                 250                 255

Ala Ser Gly Tyr Thr Phe Thr Arg Tyr Thr Met His Trp Val Lys Gln
            260                 265                 270

Arg Pro Gly Gln Gly Leu Glu Trp Ile Gly Tyr Ile Asn Pro Ser Arg
        275                 280                 285

Gly Tyr Thr Asn Tyr Asn Gln Lys Phe Lys Asp Lys Ala Thr Leu Thr
    290                 295                 300

Thr Asp Lys Ser Ser Ser Thr Ala Tyr Met Gln Leu Ser Ser Leu Thr
305                 310                 315                 320

Ser Glu Asp Ser Ala Val Tyr Tyr Cys Ala Arg Tyr Tyr Asp Asp His
                325                 330                 335

Tyr Cys Leu Asp Tyr Trp Gly Gln Gly Thr Thr Leu Thr Val Ser Ser
            340                 345                 350

Gly Gly Ser Gly Gly Ser Gly Gly Ser Gly Gly Ser Gly Gly Ser Gln
        355                 360                 365

Ile Val Leu Thr Gln Ser Pro Ala Ile Met Ser Ala Ser Pro Gly Glu
    370                 375                 380

Lys Val Thr Met Thr Cys Ser Ala Ser Ser Ser Val Ser Tyr Met Asn
385                 390                 395                 400

Trp Tyr Gln Gln Lys Ser Gly Thr Ser Pro Lys Arg Trp Ile Tyr Asp
                405                 410                 415

Thr Ser Lys Leu Ala Ser Gly Val Pro Ala His Phe Arg Gly Ser Gly
            420                 425                 430

Ser Gly Thr Ser Tyr Ser Leu Thr Ile Ser Gly Met Glu Ala Glu Asp
        435                 440                 445

Ala Ala Thr Tyr Tyr Cys Gln Gln Trp Ser Ser Asn Pro Phe Thr Phe
    450                 455                 460

Gly Ser Gly Thr Lys Leu Glu Ile Asn Ala Ala Ala Gly Ser Trp Ser
465                 470                 475                 480

His Pro Gln Phe Glu Lys
                485

<210>  5
<211>  8
<212>  PRT
<213>  artificial sequence

<220>
<223>  Tag-sequence

<400>  5

Asp Tyr Lys Asp Asp Asp Asp Lys
1               5

<210>  6
<211>  8
<212>  PRT
<213>  artificial sequence

<220>
<223>  Tag-sequence

<400>  6

Trp Ser His Pro Gln Phe Glu Lys
1               5

## Description

The present invention relates to a multifunctional, for example trifunctional, and multimeric, for example dimeric, antigen-binding molecule and its therapeutic application, for example in immunotherapy. The molecule is a Fv-antibody derivative.

Bispecific, i.e. bifunctional, antibodies can be used to engage two different therapeutic targets or perform two distinct functions. Such antibodies can be used for example to recruit an immune effector cell, e.g. T- or NK-cell, towards a particular target cell. Various antibody-fragment based molecules are known and under investigation, for example for cancer therapy.

Bifunctional and dimeric antibodies can be constructed using only antibody variable domains. For example, the linker sequence between the VH and VL domains can be made so short that they cannot intramolecularly fold over and bind one another. Such short linkers, e.g. 2-12 amino acid residues, prevent the configuration of a scFv molecule and favors intermolecular VH-VL pairings between complementary variable domains of different polypeptide chains forming a "diabody" (Holliger et al., 1993, Proc. Natl. Acad. Sci. USA 90, 6444-6448). Such diabody can be used for bifunctional antibodies, which are obtained by non-covalent association of two single-chain polypeptide fusion products, each consisting of the VH domain from one antibody connected by a short linker to the VL domain of another antibody.

WO 03/025018 discloses a bispecific and multimeric antigen-binding molecule which structure is formed by identical single-chain polypeptides with at least four binding domains. A VH and a VL domain at a terminal part of each polypeptide chain are linked by a short linker and associate intermolecularly with the corresponding VH and VL domains of another polypeptide chain, while the other VH and VL domains of each polypeptide chain bind intramolecularly to one another within the same chain resulting in an antigen-binding scFv unit. Such constructs are homodimers, i.e. they consist of identical single-chain polypeptides associated with one another.

The present invention provides a multifunctional antigen-binding polypeptide multimer.

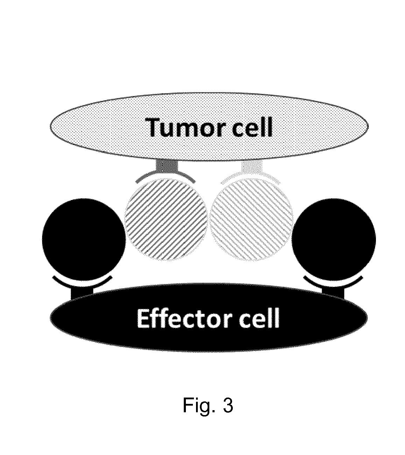

According to the invention such multifunctional antigen-binding polypeptide may be a trispecific antibody for dual targeting of tumor cells - these are trifunctional structures that can be designed to target two different targets/epitopes on the tumor cell and with the third functionality bind with high affinity to either T-cells or NK-cells. Trispecific antibodies targeting two distinct tumor epitopes and engaging T- or NK-cells lyse the tumor cells that express both targets (Figure 3). Such molecule can be generated by antibody formats consisting of four binding domains. For example, the four binding domains may be obtained by non-covalent association of two polypeptide chains.

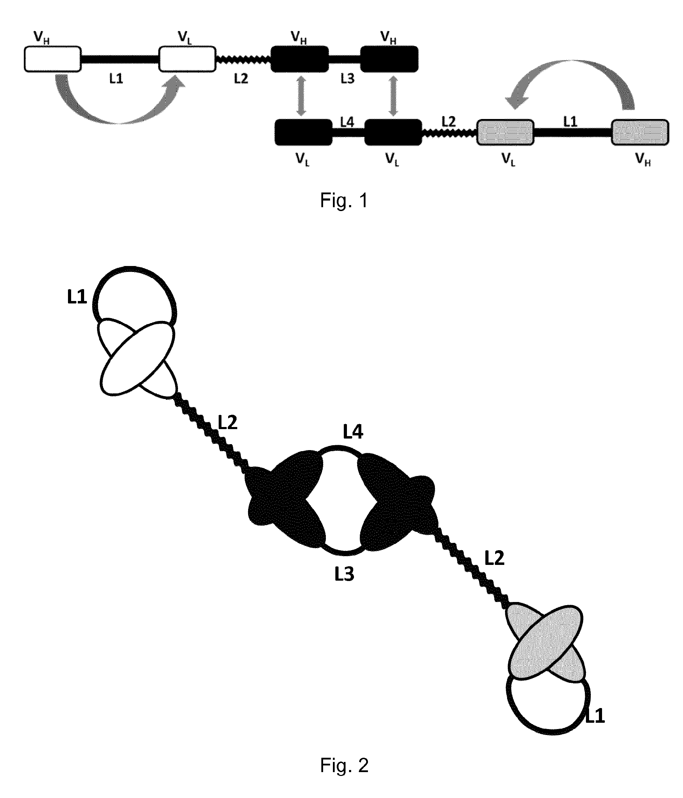

In one aspect, the present invention provides a trifunctional antigen-binding polypeptide multimer designed to target three different antigens or epitopes. Such a multimer comprises a first polypeptide and a second polypeptide. Each of the two polypeptides is a single-chain fusion peptide having at least four antibody variable domains linked one after another from the N- to the C-terminus of each polypeptide. Each of the polypeptides comprises two antibody variable domains linked by a short linker for preventing intramolecular pairing within the same polypeptide and a single-chain Fv unit having an antibody variable domain pair of the other two variable domains capable of intramolecularly forming an antigen binding site by the variable domain pair within the same polypeptide (Figure 1). The multimer is formed by non-covalent association between the two polypeptides, whereas the two antibody variable domains linked by a short linker of one polypeptide associate with the two corresponding antibody variable domains of the other polypeptide, thereby forming two additional antigen binding sites (Figure 2). Thus, the multimer comprises at least four antigen binding sites and is at least tetravalent. In a particular aspect of the invention the multimer is a dimer.

For generating such a trispecific and tetravalent antigen-binding dimer the two polypeptides have to be of different antibody variable domain compositions, because with respect to at least one of the three specificities the respective antibody variable light domain and variable heavy domain have to be inserted into different polypeptides such that one of the polypeptides contains only the variable heavy domain and the other polypeptide contains only the variable light domain for this specificity. Thus, such a dimer according to the invention is heterodimeric, because it is composed of two different polypeptides. Therefore, particular measures have to be taken for enabling a correct association of the two different polypeptides comprising antibody variable domains for three different specificities and to prevent a wrong homodimerization between two identical polypeptides. In one aspect of the invention, the inventors have obtained a correct heterodimerization between the two different, trispecific polypeptides by inserting two antibody variable heavy domains linked by a short linker in one polypeptide and inserting the two corresponding antibody variable light domains linked by a short linker into the other polypeptide. Surprisingly, only heterodimeric species of the trispecific antigen-binding polypeptide dimers have been formed.

Therefore, in one embodiment the invention provides
a trispecific antigen-binding polypeptide dimer comprising a first polypeptide and a second polypeptide, each polypeptide having at least four antibody variable domains linked one after another, and
(a) the first polypeptide comprises a first and a second antibody variable heavy domain (VH) linked with each other by a first linker preventing intramolecular pairing within the same polypeptide, for example of about 12 or less amino acid residues, and a single chain Fv antigen binding unit having a third antibody variable heavy domain (VH) linked by a second peptide linker with an antibody variable light domain (VL), said third antibody variable heavy domain (VH) and antibody variable light domain (VL) are capable to associate to a first antigen binding site, wherein the second antibody variable heavy domain (VH) is linked with the single chain Fv antigen binding unit by a third peptide linker; and
(b) the second polypeptide comprises a first and a second antibody variable light domain (VL) linked with each other by a second peptide linker preventing intramolecular pairing within the same polypeptide, for example of about 12 or less amino acid residues, and a single chain Fv antigen binding unit having a third antibody variable domain (VL) linked by a second peptide linker with an antibody variable heavy domain (VH), said third antibody variable light domain (VL) and antibody variable heavy domain (VH) are capable to associate to a second antigen binding site, wherein the second antibody variable light domain (VL) is linked with the single chain Fv antigen binding unit by a third peptide linker; and
(c) the first and the second antibody variable heavy domain (VH) of the first polypeptide associate with the first and the second antibody variable light domain (VL) of the second polymer to two additional, i.e. a third and forth, antigen binding sites, whereas in a preferred embodiment the first antibody variable heavy domain (VH) of the first polypeptide associates with the second antibody light chain region (VL) of the second polypeptide to a third antigen binding site and the second antibody variable heavy domain (VH) of the first polypeptide associates with the first antibody variable light domain (VL) of the second polypeptide to a fourth antigen binding site.

A trispecific antigen-binding polypeptide dimer is formed, when two of said four antigen binding sites are specific for the same antigen (Figure 1 and Figure 2).

Such a trispecific dimer recognizes three different specificities, and can target, for example, two different antigens or epitopes on a target cell and with the third functionality, i.e. specificity, bind, for example, to an immune effector cell such as, for example, a T- or a NK-cell.

In one aspect, the trispecific dimer according to the invention can be used as an anticancer agent for the therapeutic concept of redirecting the cytotoxic potential of immune effector cells to lyse tumor cells. Preferably, the trispecific dimer targets bifunctionally a tumor cell, i.e. has specifities for two distinct epitopes on a tumor cell and engages an immune effector cell, e.g. T- or a NK-cell, with the third specificity. The trispecific dimer can bind two different tumor antigens with high specificity and can have high affinity to activating receptor on the immune effector cell. The binding to two distinct tumor epitopes leads to an increase in targeting specificity and to an extension of the therapeutic window by reducing off-target toxicities. Importantly, despite the structural complexity, such trispecific dimer according to the invention is stable and can be manufactured efficiently.

Exemplified herein are trispecific dimers according to the invention containing CD3-, CD19- and CD30-binding domains. Heterodimeric species are obtained from cell culture supernatant, the trispecific dimers exhibit excellent stability and exhibit higher potency on double-positive target cell lines when compared to respective single-positive target cell line.

The multifunctional, in particular trispecific, dimer according to the invention can be utilized in different ways.

For example, the antibody variable domains may be arranged within a polypeptide such that the two antibody variable domains associating with the two corresponding antibody variable domains of the other polypeptide may be positioned, for example, at the N-terminus or the C-terminus of the polypeptide. These two antibody variable domains may have the same specificity or distinct specificities. For example, both may be specific for the same immune effector cell or have distinct specificities for two antigens on a tumor cell.

Further, the two antibody variable domains forming the single-chain Fv unit may be, for example, in the order VH-VL or VL-VH in the direction from the N- to the C-terminus of the polypeptide. The single-chain Fv units of the two dimerized polypeptides may have the same or different specificities. For example, if the two antibody variable domains associating with the two corresponding antibody variable domains of the other polypeptide have the same specificity, the single-chain Fv units of the two polypeptides have different specificities for achieving a trispecific dimer.

Thus, the at least four antibody variable domains may be arranged, for example, such that the two antibody variable domains associating with the two corresponding antibody variable domains of the other polypeptide are specific for an immune effector cell and the single-chain Fv units of the two polypeptides have specificities for two distinct tumor antigens or the two antibody variable domains associating with the two corresponding antibody variable domains of the other polypeptide are specific for distinct tumor antigens and both single-chain Fv units of the two polypeptides have the same specificity for an immune effector cell.

The "antigen-binding polypeptide dimer" refers to an immunoglobulin derivative with multivalent antigen-binding properties, preferably having at least four antigen-binding sites. Each antigen-binding site is formed by an antibody, i.e. immunoglobulin, variable heavy domain (VH) and an antibody variable light domain (VL) having binding specificity to the same epitope. The epitope may be on the same or different antigens. Preferably, the antigen-binding polypeptide dimer according to the invention is devoid of immunoglobulin constant domains or fragments thereof.

Preferably, the antigen-binding polypeptide is a "dimer" which term refers to a complex of a first and a second polypeptide monomer. In one aspect the antigen-binding polypeptide dimer is a "heterodimer" which term means that the antigen-binding polypeptide is composed of two different polypeptide monomers that are encoded by two distinct polynucleotides.

Preferably, in the antigen-binding dimer the first and the second polypeptides are non-covalently associated with each other, in particular with the proviso that there is no covalent bound between the first and second polypeptide. However, if desired, the two polypeptides may be additionally stabilized by at least one covalent linkage, e.g. by a disulfide bridge between cysteine residues of different polypeptides.

The term "polypeptide" refers to a polymer of amino acid residues linked by amide bonds. The first and the second polypeptides are, preferably, single chain fusion proteins which are not branched. In each of the first and second polypeptides the variable antibody domains are linked one after another. The first and the second polypeptide may have contiguous amino acid residues in addition N-terminal and/or C-terminal. For example, the polypeptide may contain a Tag sequence, preferably at the C-terminus which might be useful for the purification of the polypeptide. Examples of a Tag sequence are a His-Tag, e.g. a His-Tag consisting of six His-residues, a FLAG, e.g. a DYKDDDDK octapeptide (SEQ ID NO:5) or STREP® II, e.g a WSHPQFEK octapeptide (SEQ ID NO:6). Preferably, different Tag sequences are used for the first and the second polypeptide.

The length of the linkers influences the flexibility of the antigen-binding polypeptide dimer. The desired flexibility of the antigen-binding polypeptide dimer depends on the target antigen density and the acessibility of the target antigen, i.e. epitopes. Longer linkers provide a more flexible antigen-binding polypeptide dimer with more agile antigen-binding sites. The effect of linker length on the formation of dimeric antigen-binding polypeptides is described, for example, in Todorovska et al., 2001 Journal of Immunological Methods 248:47-66; Perisic et al., 1994 Structure 2:1217-1226; Le Gall et al., 2004, Protein Engineering 17:357-366 and WO 94/13804.

According to the invention it is preferred that the length of the first peptide linker of the first and second antibody variable heavy domains of the first polypeptide and the first and second antibody variable light domains of the second polypeptide is such that the domains of the first polypeptide can associate intermolecularly with the domains of the second polypeptide to form the dimeric antigen-binding polypeptide. Such linkers are "short", i.e. consist of 0, 1, 2, 3, 4, 5, 6, 7, 8, 9, 10, 11 or about 12 amino acid residues. In the case of 0 amino acid residues the linker is a peptide bond. Such short linkers favor the correct dimerization of the first with the second polypeptide by binding and forming antigen-binding sites between antibody variable light domains and antibody variable heavy domains of different polypeptides. Shortening the linker to about 12 or less amino acid residues generally prevents adjacent domains of the same polypeptide chain from interacting with each other. In an embodiment of the invention these linkers consist of about 3 to about 10, for example 7 contiguous amino acid residues. Besides, it is in principle possible that two polypeptides having a linker with more than 12 amino acid residues between the variable antibody domains correctly dimerize with one another (see for example Le Gall et al., 2004, Protein Engineering 17:357-366).

For the single-chain Fv units of the polypeptides the second peptide linker is long and flexible (in general consisting of about 12 or more amino acid residues) for folding intramolecularly head-to-tail and forming the single-chain antigen-binding unit. Additional amino acid residues provide extra flexibility. For example this linker between the VH and VL or VL and VH of the single-chain Fv unit in the polypeptide may consist of about 12 to about 35, in particular from 15 to 25 contiguous amino acid residues.

The third peptide linker of the polypeptide for linking the single-chain Fv unit with the other two antibody variable domains which associate with the corresponding variable domains of the other polypeptide may be, for example, from 5 to 30, preferably at least 6, 7, 8, 9, 10, 11, 12, 13. 14 or 15 contiguous amino acid residues. The length can be adapted dependent on the desired degree of flexibility within the antigen-binding polypeptide dimer.

Regarding the amino acid composition of the peptide linkers, peptides are selected that do not interfere with the dimerization of the first and second polypeptides. For example, linkers comprising glycine and serine residues generally provide protease resistance. The amino acid sequence of the linkers can be optimized, for example, by phage-display methods to improve the antigen binding and production yield of the antigen-binding polypeptide dimer. In an embodiment (G₂S)ₓ peptide linkers are used.

In a certain aspect of the invention at least one, preferably all, antibody variable domains are fully human, humanized or chimeric domains. Humanized antibodies can be produced by well-established methods such as, for example CDR-grafting (see, for example, Antibody engineering: methods and protocols / edited by Benny K.C. Lo; Benny K.C. II Series: Methods in molecular biology (Totowa, N.J.)). Thus, a skilled person is readily able to make a humanized or fully human version of antigen-binding molecules and variable domains from non-human, e.g. murine or non-primate, sources with the standard molecular biological techniques known in the art for reducing the immunogenicity and improving the efficiency of the antigen-binding molecule in a human immune system. In a preferred embodiment of the invention all antibody variable domains are humanized or fully human; most preferred, the antigen-binding polypepdtide dimer according to the invention is humanized or fully human. The term "fully human" as used herein means that the amino acid sequences of the variable domains and the peptides linking the variable domains in the first and second polypeptides originate or can be found in humans. In certain embodiments of the invention the variable domains may be human or humanized but not the peptides linking the antibody variable domains.

In an aspect of the invention the antigen-binding polypeptide dimer is trispecific comprising a first specificity for an effector cell and a second and a third specificity for a target cell different from the effector cell. Such antigen-binding polypeptide dimer is able to cross-link two cells and can be used to direct effector cells to a specific target.

"Effector cells" are cells of the immune system which can stimulate or trigger cytotoxicity, phagocytosis, antigen presentation, cytokine release. Such effector cells are, for example but not limited to, T cells and natural killer (NK) cells. Examples of suitable soecificities for effector cells include but are not limited to CD3 or CD3 subunits such as CD3ε for T cells and CD16 for NK cells. Such cell surface molecules, of effector cells are suitable for mediating cell killing upon binding of a multispecific, e.g. trispecific, antibody to such cell surface molecule and, thereby, inducing cytolysis.

"Target cells" are the sites to which the effector cells should be redirected to induce or trigger the respective biological, e.g. immune, response. Examples of target cells may be tumor cells.

In a trispecific embodiment of the invention the antigen-binding polypeptide dimer is bispecific for two distinct antigens on a tumor cell and additionally specific for an effector cell, in particular a T cell or a NK cell. Suitable specificities for tumor cells may be tumor antigens and cell surface antigens on the respective tumor cell, for example specific tumor markers. Such a trispecific antigen-binding dimer binds bifunctionally to a tumor cell and to the immune effector cell thereby triggering the cytotoxic response induced by the T cell or the NK cell. The term "tumor antigen" as used herein comprises tumor associated antigen (TAA) and tumor specific antigen (TSA). A "tumor associated antigen" (TAA) as used herein refers to a protein which is present on tumor cells, and on normal cells during fetal life (once-fetal antigens), and after birth in selected organs, but at much lower concentration than on tumor cells. A TAA may also be present in the stroma in the vicinity of the tumor cell but expressed at lower amounts in the stroma elsewhere in the body. In contrast, the term "tumor specific antigen" (TSA) refers to a protein expressed by tumor cells. The term "cell surface antigen" refers to any antigen or fragment thereof capable of being recognized by an antibody on the surface of a cell.

Examples of specificities for tumor cells include but are not limited to CD19 and CD30.

The specificity for an effector cell includes but is not limited to CD3 for T-cells, and CD16 for NK-cells.

CD3 antigen is associated with the T-cell receptor complex on T-cells. In the case where specificity for an effector cell is CD3, the binding of the antigen-binding molecule according to the invention to CD3 triggers the cytotoxic activity of T-cells. By binding of the antigen-binding molecule to CD3 and to a target cell, e.g. tumor cell, cell lysis of the target cell may be induced.

The CD16 (FcγRIII) antigen is a receptor expressed on the surface of NK cells. NK cells possess an inherent cytoloytic activity and by binding of the antigen-binding molecule according to the invention to CD16 the cytotoxic activity of NK cell towards the target cell can be triggered. Human CD16 exists as two isoforms, CD16A (FcγRIIIA) and CD16B(FcγRIIIB). CD16A is expressed on macrophages, mast cells, and NK cells. CD16B is present on polymorphonuclear granulocytes (PMN). In one embodiment of the invention the antigen-binding polypeptide dimer is specific for CD16A.

Antigen-binding molecules according to the invention, wherein the tumor specificity is towards CD19 antigen may be used for immunotherapy of B-cell malignancies, because the CD19 antigen is expressed on virtually all B-lineage malignancies from lymphoblastic leukemia (ALL) to non-Hodgkin's lymphoma (NHL).

Antigen-binding molecules according to the invention wherein the tumor specificity is towards CD30 may be particularly useful in treating Hodgkin's disease.

For increasing serum-half life of the antigen-binding molecules according to the invention in the body, the antigen-binding molecule, if desired, may be fused to albumin or pegylated, sialylated or glycosylated (see, for example, Stork et al., 2008, J. Biol. Chem., 283:7804-7812).

The antigen-binding polypeptide according to any one of the embodiments described here previously may be produced by expressing polynucleotides encoding the individual polypeptide chains which associate with each other to form the antigen-binding polypeptide dimer. Therefore, a further embodiment of the invention are polynucleotides, e.g. DNA or RNA, encoding the polypeptide chains of the antigen-binding dimer as described herein above.

The polynucleotides may be constructed by methods known to the skilled person, e.g. by combining the genes encoding the at least four antibody variable domains either separated by peptide linkers or directly linked by a peptide bound of the first and second polypeptide, into a genetic construct operably linked to a suitable promoter, and optionally a suitable transcription terminator, and expressing it in bacteria or other appropriate expression system such as, for example CHO cells. Depending on the vector system and host utilized, any number of suitable transcription and translation elements, including constitutive and inducible promoters, may be used. The promoter is selected such that it drives the expression of the polynucleotides in the respective host cell.

The polynucleotides may be inserted into vectors, preferably expression vectors, which represent a further embodiment of the invention. These recombinant vectors can be constructed according to methods well known to the person skilled in the art.

A variety of expression vector/host systems may be utilized to contain and express the polynucleotides encoding the polypeptide chains of the present invention. Examples for expression vectors for expression in *E.coli* is pSKK (LeGall et al., J Immunol Methods. (2004) 285(1):111-27) or pcDNA5 (Invitrogen) for the expression in mammal cells.

Thus, the antigen-binding polypeptide dimer as described herein may be produced by introducing a vector encoding the polypeptide chains as described above into a host cell and culturing said host cell under conditions whereby the polypeptide chains are expressed, may be isolated and, optionally, further purified.

In a further embodiment of the invention compositions comprising a antigen-binding polypeptide dimer or polynucleotides as described herein above and at least one further component are provided.

The invention further provides a method wherein the antigen-binding polypeptide dimer as described herein above is administered in an effective dose to a subject, e.g., patient, for the treatment of cancer (e.g. non-Hodgkin's lymphoma; chronic lymphocytic leukaemia). The antigen-binding polypeptide dimer can be used as a medicament.

A skilled person will readily be able without undue burden to construct and obtain the antigen-binding polypeptide dimer described herein by utilizing established techniques and standard methods known in the art, see for example Sambrook, Molecular Cloning A Laboratory Manual, Cold Spring Harbor Laboratory (1989) N.Y.; The Protein Protocols Handbook, edited by John M. Walker, Humana Press Inc. (2002); or Antibody engineering: methods and protocols / edited by Benny K.C. Lo; Benny K.C. II Series: Methods in molecular biology (Totowa, N.J.)).

### Brief description of the figures:

Figure 1 shows a first and a second polypeptide for forming a trifunctional, i.e. trispecific, antigen-binding polypeptide dimer according to the invention. The first polypeptide has four antibody variable domains VH, VL, VH, VH linked one after another. The first and the second VH antibody variable domains (black) have the same first specificity and are linked by a short linker L3 for preventing intramolecular pairing within the same polypeptide and a single-chain Fv unit having an antibody variable domain pair of the other third variable antibody domain VL and the fourth variable antibody domain VH (white) linked by a second linker L1 capable of intramolecularly forming an antigen binding site of a second specificity by the variable domain pair within the same polypeptide. The second antibody variable domain VH and the third antibody variable domain VL of different specificities are linked by a third linker L2.
   The second polypeptide has four antibody variable domains VL, VL, VL, VH linked one after another. The first and the second VL antibody variable domains (black) have the same first specificity and are linked by a short linker L4 for preventing intramolecular pairing within the same polypeptide and a single-chain Fv unit having an antibody variable domain pair of the other third variable antibody domain VL and the fourth variable antibody domain VH having a third specificity (grey) and are linked by a second linker L1 capable of intramolecularly forming an antigen binding site by the variable domain pair within the same polypeptide. The second antibody variable domain VL and the third antibody variable domain VL of different specificities are linked by a third linker L2.
Figure 2 shows the antigen-binding polypeptide dimer formed by non-covalent association between the two polypeptides of Figure 1, whereas the two antibody variable VH domains linked by a short linker of the first polypeptide associate with the two corresponding antibody variable VL domains of the second polypeptide, thereby forming two antigen binding sites having the same specificity (black), whereas the second specificity is provided by the single chain Fv unit of the first polypeptide (white) and the third specificity is provided by the single chain Fv unit of the second polypeptide (grey).
Figure 3 shows a trifunctional antigen-binding polypeptide according to the invention which is trispecific antibody for dual targeting of tumor cells. The antibody, i.e. antigen-binding polypeptide, is designed to target two different targets/epitopes on the tumor cell and with the third functionality bind with high affinity to an effector cell. The antigen-binding polypeptide consists of four antigen binding sites, wherein the two central antigen binding sites bind to two different antigens on the tumor cell and the two peripheral antigen binding sites bind to the effector cell.

The examples below further illustrate the invention without limiting the scope of the invention.

### Example 1

### DNA constructs:

The plasmid DNA encoding the polypeptide chains are generated by DNA engineering or by gene synthesis and sequencing. The expression constructs for transient or stable transfection of mammalian cells are based on the eukaryotic expression vector pCDNA5/FRT (Life Technologies) and comprise the product gene of interest under the control of a viral or ubiquitous promoter, as well as a Hygromycin resistance cassette as a selection marker. For purification and analytics, the product chains are expressed with His-tag, FLAG-tag or StrepII-tag.

### Cell Lines and Cell Cultivation:

Flp-In CHO cells (Life Technologies), a derivative of CHO-K1 Chinese Hamster ovary cells (ATCC, CCL-61) (Kao and Puck, 1968), are cultured in Ham's F-12 Nutrient Mix supplemented with L-Glutamine, 10% FCS and 100 µg/ml Zeocin. Adherent cells are detached with 0.25% Trypsin-EDTA and subcultured according to standard cell culture protocols.

For adaptation to growth in suspension, cells are detached from tissue culture flasks and placed in serum-free medium for subsequent incubation in shake flasks (Corning) at 37°C, 5% CO₂ and 120 rpm. The standard medium for the culture of suspension-adapted Flp-In CHO cells is HyClone CDM4 CHO (Thermo Scientific) supplemented with L-Glutamine (Life Technologies), HT Supplement (Life Technologies), Penicillin/Streptomycin (Life Technologies) and 100 µg/ml Zeocin (Life Technologies). Suspension-adapted cells are subcultivated every 2-3 days with seeding densities of 2E+6 to 3E+6 cells/ml. The cell concentration and viability is determined in all cultures using the trypan blue exclusion method. Cells are cryopreserved in medium with 10% DMSO and tested negative for Mycoplasma using MycoAlert Mycoplasma detection Kit (Lonza).

### Generation of stably transfected cell pools:

Recombinant Flp-In CHO cell lines stably expressing tri-specific candidate antibodies, are generated by transfection of suspension-adapted cells. For this, cells are placed in standard medium without Zeocin one day prior to co-transfection with expression plasmids (2.5 µg) encoding the protein of interest (pcDNA5/FRT) and the Flp recombinase (pOG44, Life Technologies) using Polyethylenimine (PEI). In brief, vector DNA and transfection reagent are mixed at a DNA:PEI mass ratio of 1:3 in a total of 100 µL OptiMEM I medium (Life Technologies) and incubated for 10 minutes before addition to 2E+6 Flp-In CHO cells suspended in 1ml CHO-S-SFMII medium (Life Technologies). Following 24h incubation, selection for stably transfected cells is started by addition of 500µg/mL Hygromycin B subsequent to diluting cultures to a density of 0.1E+6 viable cells/mL in CHO-S-SFMII medium and seeding in T75 culture flasks. Flp recombinase mediates the insertion of the Flp-In expression construct into the genome at the integrated FRT site through site-specific DNA recombination (O' Gorman et al 1991). During selection viable cell densities are measured twice a week, and cells are centrifuged and resuspended in fresh selection medium at a maximal density of 0.1 E+6 viable cells/mL.Cell pools stably expressing recombinant protein products are recovered after approximately 3 weeks of selection at which point cells are transferred to standard culture medium in shake flasks. Expression of recombinant secreted proteins is confirmed by protein gel electrophoresis of cell culture supernatants using Criterion Stain-Free (Bio-Rad) technology. Stable cell pools are cryopreserved in medium containing 50% ProFreeze (Lonza) and 7.5% DMSO.

### Production of recombinant protein in Fed-batch CHO cell suspension cultures :

Recombinant proteins are produced in 10-day fed-batch cultures of stably transfected CHO cell lines by secretion into the cell culture supernatant. For this, cell pools stably expressing the product of interest are seeded at starting densities of 6E+5 cells/mL in standard culture medium in polycarbonate Erlenmeyer flasks with gas permeable caps (Corning) and incubated at 37°C and 5% CO2 with agitation at 140 rpm. During fed-batch culture, media is supplemented with 40 mL/L ActiCHO Feed A (PAA) and 4 mL/L ActiCHO Feed B (PAA) on day 0 (starting day), and with double amounts on day 3, 5, and 7. Cell culture supernatants are harvested after 10 days at culture viabilities of typically >75%. Samples are collected from the production cultures every other day prior to feeding and cell density and viability is assessed. On the day of harvest, cell culture supernatants are cleared by centrifugation and vacuum filtration (0.22 µm) using Millipore Express PLUS Membrane Filters (Millipore) before further use.

### Determination of expression titer :

Protein expression titers and product integrity in cell culture supernatants (CCS) are analysed by SDS-PAGE using the Criterion Stain-Free gel imaging system (Bio-Rad) on days 7 and 10 (before and after 0.22 µm filtration). Product titers are determined semi-quantitatively by comparison with a reference protein of known concentration.

### Purification of trispecific antigen-binding polypeptides :

His-tagged products are purified from CHO cell culture supernatants in a two-step procedure comprising Ni-NTA- and preparative size-exclusion chromatography. First, supernatants are cleared by vacuum filtration (0.22 µm) and adjusted to 5 mM imidazole before loading onto HisTrap FF chromatography column (GE Healthcare) equilibrated in IMAC Buffer A at a flow rate of 5 mL/min. Columns are subsequently washed with 5 CV IMAC Buffer A and 10 CV of a mixture of IMAC Buffer A and IMAC Buffer B (7%). His-tagged products are then eluted by sequential washing with 10 CV 30% IMAC Buffer B and 5 CV 100% IMAC Buffer B at the same flow rate. 2.5 mL eluate fractions are collected and protein content and purity is assessed by subjecting each fraction to one-dimensional SDS-PAGE followed by visualization of protein using Criterion Stain-Free technology (Bio-Rad). Product containing fractions are pooled and concentrated by ultrafiltration. Subsequently, concentrated samples are purified by gel filtration using a HiLoad 26/600 Superdex 200 pg (GE Healthcare) column and eluted in SEC Buffer (20 mM Tris-HCl, 100 mM NaCl, pH 7.5) at 2.5 mL/min. Fractions containing the purified product, as determined by comparison of elution volumes with column retention of molecular weight marker proteins (GE Healthcare), are collected and pooled. After a final buffer exchange (10 mM sodium acetate, pH 5.0) using PD-10 desalting columns (GE Healthcare) samples are concentrated to 1.0 - 1.5 mg/mL by ultrafiltration as described above. Purity and homogeneity (typically >90%) of final samples are assessed by Criterion Stain-Free gel visualization of proteins after reducing and non-reducing SDS-PAGE as described above, in selected cases followed by immunoblotting with specific antibodies and by analytical SEC, respectively. Purified proteins are stored as aliquots at -80°C until further use.

### Examples 2 CD3xCD19xCD30 trispecific molecules

Antigen-binding polypeptide dimers containing CD3-, CD19- and CD30-antibody variable binding domains originating from the antibodies OKT3, HD37 and HRS3, respectively are produced according to Example 1:
Trispec 1:
   VH(CD3)-(G₂S)₂- VH(CD3) -(G₂S)₃-VH(CD30)-(G₂S)₅-VL(CD30)-His₆ (SEQ ID NO:1)
   VL(CD3)- (G₂S)₂-VL(CD3)-(G₂S)₃-VH(CD19)-(G₂S)₅-VL(CD19)-FLAG (SEQ ID NO:2)
Trispec 2:
   VH(CD30)-(G₂S)₂-VH(CD19)-(G₂S)₃-VH(CD3)-(G₂S)₅-VL(CD3)-His₆ (SEQ ID NO:3)
   VL(CD19)-(G₂S)₂-VL(CD30)- (G₂S)₃-VH(CD3)-(G₂S)₅-VL(CD3)-FLAG (SEQ ID NO:4)

First Linker (L3/L4) = (G₂S)₂, Second Linker (L1) = (G₂S)₅, Third Linker (L2) = (G₂S)₃ Immunoprecipitation of Trispec 1 and Trispec 2 show that only heterodimeric species of the antigen-binding polypeptide dimer are detected. Trispec 1 and Trispec 2 exhibit excellent stability at 40°C after 7 days and at pH 3.5 after 1 h.

### Example: Assessment of cytotoxic activity mediated by trispecific antibodies

### Study procedures

### Isolation of PBMC from buffy coats and enrichment of T cells:

PBMCs are isolated from buffy coats by density gradient centrifugation. T cells are enriched from the PBMC population using the EasySep™ Human T Cell Enrichment Kit for the immunomagnetic isolation of untouched human T cells and the Big Easy EasySep™ Magnet according to the manufacturer's instructions.

### FACS-based cytotoxicity assay:

T cells that are used as effector cells are characterized by flow cytometry as described.

Target cells (MEC-1: DSMZ, cat.: ACC 497; NALM-6: DSMZ, cat.: ACC 128) are cultured under standard conditions as described below. For the cytotoxicity assay target cells are harvested, washed twice with RPMI 1640 medium without FCS, and resuspended in diluent C provided in the PKH67 Green Fluorescent Cell Linker Mini Kit to a density of 2x10⁷/mL. The cell suspension is then mixed with the equal volume of a double-concentrated PKH67-labeling solution (e.g. 1 µL PKH67 in 250 µL diluent C) and incubated according to the manufacturer's instructions. The staining reaction is stopped. After washing the labeled target cells with complete RPMI medium, cells are counted and resuspended to a density of 2x10⁵/mL in complete RPMI medium.

2x10⁴ target cells are then seeded together with T cells at and the indicated antibodies in individual wells. Spontaneous cell death and killing of targets by effectors in the absence of antibodies are determined.

After incubation, cultures are washed once with FACS buffer and then resuspended in 150 µL FACS buffer supplemented with 2 µg/mL PI. The absolute amount of living target cells that are characterized by a positive green PKH67 staining but are negative for the PI staining are measured using a Beckman-Coulter FC500 MPL flow cytometer (Beckman-Coulter) or a Millipore Guava EasyCyte flow cytometer (Merck Millipore).

Based on the measured remaining living target cells, the percentage of specific cell lysis is calculated according to the following formula: [1-(number of living targets ₍ₛₐₘₚₗₑ₎) / (number of living targets ₍ₛₚₒₙₜₐₙₑₒᵤₛ₎)] x 100%. Sigmoidal dose response curves and EC₅₀ values are calculated by non-linear regression/4-parameter logistic fit using the GraphPad Prism software (GraphPad Prism version 6.00 for Windows, GraphPad Software, La Jolla California USA).

### Statistical analysis

The lysis values obtained for a given antibody concentration are determined and analysed by sigmoidal dose-response/4 parameter logistic fit analysis using the Prism software (GraphPad Prism version 6.00 for Windows, GraphPad Software, La Jolla California USA) and used to calculate EC₅₀ values, and mean and SD of replicates of percentage lysis.

### Results:

Trispec 1 and Trispec 2 exhibit higher cytotoxic potency on double-positive cell lines (CD19⁺ and CD30⁺) when compared to the respective single-positive cell lines.

## Claims

1. A trispecific antigen-binding polypeptide dimer comprising a first polypeptide and a second polypeptide, each polypeptide having at least four antibody variable domains linked one after another, wherein
(a) the first polypeptide comprises a first and a second antibody variable heavy domain (VH) linked with each other by a linker of about 12 or less amino acid residues and a single chain Fv antigen binding unit having a third antibody variable heavy domain (VH) linked with an antibody variable light domain (VL), said third antibody variable heavy domain (VH) and antibody variable light domain (VL) are capable to associate to a first antigen binding site, wherein the first or second antibody variable heavy domain (VH) is linked with the single chain Fv antigen binding unit by a peptide linker;
(b) the second polypeptide comprises a first and a second antibody variable light domain (VL) linked with each other by a linker of about 12 or less amino acid residues and a single chain Fv antigen binding unit having a third antibody variable light domain (VL) linked with an antibody variable heavy domain (VH), said third antibody variable light domain (VL) and antibody variable heavy domain (VH) are capable to associate to a second antigen binding site, wherein the first or second antibody variable light domain (VL) is linked with the single chain Fv antigen binding unit by a peptide linker;
(c) the first antibody variable heavy domain (VH) of the first polypeptide associates with the second antibody variable light domain (VL) of the second polypeptide to a third antigen binding site;
(d) the second antibody variable heavy domain (VH) of the first polypeptide associates with the first antibody variable light domain (VL) of the second polypeptide to a fourth antigen binding site; and
(e) two of said four antigen binding sites are specific for the same antigen.

2. The antigen-binding polypeptide dimer according to claim 1, wherein the first and the second variable heavy domains of the first polypeptide and the first and the second variable light domains of the second polypeptide are linked by a linker having 3 to 9 amino acid residues.

3. The antigen-binding polypeptide dimer according to claim 1 or 2, wherein the second variable heavy domain and the single chain Fv unit of the first polypeptide and the second variable light domain and the single chain Fv unit of the second polypeptide are linked with a linker having 2 to 35 amino acid residues.

4. the antigen-binding polypeptide dimer according to anyone of claims 1 to 3, wherein the third variable heavy domain and the variable light domain of the single chain Fv unit of the first polypeptide and third variable light domain and variable heavy domain of the single chain Fv unit of the second polypeptide are linked with a linker having 12 or more amino acid residues.

5. The antigen-binding polypeptide dimer according to anyone of claims 1 to 4, wherein the first and second antigen binding sites of the two single chain Fv units are specific for the same antigen.

6. The antigen-binding polypeptide dimer according to anyone of claims 1 to 4, wherein the third and the fourth antigen binding sites formed by association between the first and the second polypeptide are specific for the same antigen.

7. The antigen-binding polypeptide dimer according to anyone of claims 1 to 6, wherein the two antigen binding sites specific for the same antigen are specific for an antigen presented on a T-cell or natural killing (NK) cell.

8. The antigen-binding polypeptide dimer according to claim 7, wherein the antigen is CD3 or CD16.

9. The antigen-binding polypeptide dimer according to claim 7 or 8, wherein the other two binding sites are specific for two different antigens on the same cell.

10. The antigen-binding polypeptide dimer according to claim 9, wherein the cell is a tumor cell.

11. The antigen-binding polypeptide dimer according to claim 10, wherein the two different antigens are selected from the group consisting of CD19 and CD30.

12. The antigen-binding polypeptide dimer according to anyone of claims 1 to 11, wherein the antigen-binding polypeptide is specific for CD3, CD19 and CD30.

13. A vector encoding an antigen-binding polypeptide dimer according to anyone of claims 1 to 12.

14. A host cell transformed by an vector according to claim 13.

15. An antigen-binding polypeptide dimer according to claim 12 for use in tumor therapy.
